# FASCICULE DE BREVET EUROPEEN

(11) **EP 1 221 952 B1**
(45) Date de publication et mention de la délivrance du brevet: **11.05.2005**
(21) Numéro de dépôt: 00966200.8
(22) Date de dépôt: 27.09.2000
(51) Int. Cl.: A61K 31/454, A61P 9/10, A61P 25/00, A61P 25/28

(54) **UTILISATION D'UN ANTAGONISTE DES RECEPTEURS AUX CANNABINOiDES CENTRAUX POUR LA PREPARATION DE MEDICAMENTS**
VERWENDUNG VON EINEM ANTAGONIST DER ZENTRALEN KANNABINOIDREZEPTOREN ZUR HERSTELLUNG VON ARZNEIMITTELN
USE OF CENTRAL CANNABINOID RECEPTOR ANTAGONIST FOR PREPARING MEDICINES

(30) Priorité: 01.10.1999 FR 9912415
(43) Date de publication de la demande: 17.07.2002
(73) Titulaire: Sanofi-Aventis, 75013 Paris (FR)
(72) Inventeur: BOURRIE, Bernard, F-34980 Saint Gély du Fesc (FR); CASELLAS, Pierre, F-34090 Montpellier (FR)
(74) Mandataire: Thouret-Lemaitre, Elisabeth
(86) Numéro de dépôt international: PCT/FR2000/002662
(87) Numéro de publication internationale: WO 2001/024798

(56) Documents cités:
- EP-A- 0 656 354
- EP-A- 0 860 168
- WO-A-00/15609
- WO-A-00/46209
- WO-A-96/02248
- US-A- 5 624 941
- PERTWEE R G: "Pharmacology of cannabinoid receptor ligands." CURRENT MEDICINAL CHEMISTRY, (1999 AUG) 6 (8) 635-64. REF: 171, XP000923352
- CONSROE P.: "Brain cannabinoid systems as targets for the therapy of neurological disorders." NEUROBIOLOGY OF DISEASE, (1998) 5/6 (534-551)., XP000920684
- NAGAYAMA T ET AL: "Cannabinoids and neuroprotection in global and focal cerebral ischemia and in neuronal cultures." JOURNAL OF NEUROSCIENCE, (1999 APR 15) 19 (8) 2987-95., XP000920687
- MOLINA-HOLGADO F. ET AL: "The endogenous cannabinoid anandamide potentiates interleukin-6 production by astrocytes infected with Theiler's murine encephalomyelitis virus by a receptor-mediated pathway." FEBS LETTERS, (1998) 433/1-2 (139-142)., XP000914617

## Description

La présente invention concerne une nouvelle utilisation d'un antagoniste des récepteurs aux cannabinoïdes centraux dits récepteurs CB₁.

Plus particulièrement, l'invention se rapporte à l'utilisation d'un antagoniste des récepteurs CB₁ pour la préparation de médicaments destinés à la prévention et au traitement des pathologies neuroinflammatoires, en particulier les maladies entraînant une démyélinisation telles que la sclérose en plaques ou le syndrome de Guillain-Barré par exemple, ainsi que les encéphalites virales, les accidents vasculaires cérébraux ou les traumatismes crâniens.

Les effets des cannabinoïdes sont dûs à une intéraction avec des récepteurs spécifiques de haute affinité couplés aux protéines G. Deux types de récepteurs sont actuellement décrits : les récepteurs CB₁, présents majoritairement au niveau du système nerveux central (Devane et al., Molecular Pharmacology, 1988, 34, 605-613) et les récepteurs CB₂ présents dans le système immunitaire (Nye et al., The Journal of Pharmacology and Experimental Therapeutics, 1985, 234, 784-791 ; Kaminski et al., 1992, Molecular Pharmacology, 42, 736-742 ; Munro et al., Nature, 1993, 365, 61-65).

Il est connu que le cannabis peut réduire ou supprimer certains symptômes associés à la sclérose en plaques tels que la spasticité musculaire et la douleur, (CNS Drugs, 1999, 11/5, 327-334).

Par ailleurs, il est décrit que des composés actifs sélectivement sur les récepteurs CB₂ aux cannabinoïdes peuvent être utilisées dans le traitement des maladies inflammatoires d'origine immunitaire (demande de brevet WO 98/31227).

L'utilisation d'agonistes des récepteurs CB₁ aux cannabinoïdes est citée pour la prévention et le traitement de maladies neurodégénératives (demande de brevet WO 98/439100).

Le N-pipéridino-5-(4-chlorophényl)-1-(2,4-dichlorophényl)-4-méthylpyrazole-3-carboxamide, ci-après dénommé composé A, de formule : 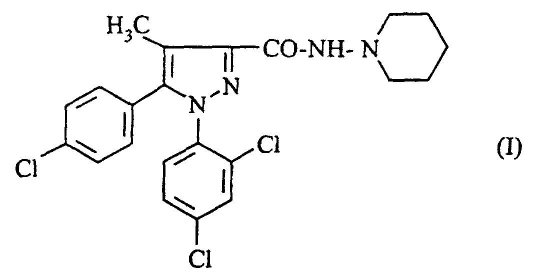 ses sels pharmaceutiquement acceptables et leurs solvats, sont décrits dans le brevet européen EP-656 354, comme antagonistes des récepteurs centraux CB₁ aux cannabinoïdes.

On a maintenant trouvé que l'administration d'un antagoniste des récepteurs CB₁ comme le composé A, ses sels pharmaceutiquement acceptables ou leurs solvats, est utile pour la prévention et le traitement des pathologies neuroinflammatoires, en particulier les maladies entraînant une démyélinisation telles que la sclérose en plaques ou le syndrome de Guillain-Barré par exemple, ainsi que les encéphalites virales, les accidents vasculaires cérébraux ou les traumatismes crâniens.

Selon un de ses aspects, la présente invention concerne l'utilisation du N-pipéridino-5-(4-chlorophényl)-1-(2,4-dichlorophényl)-4-méthylpyrazole-3-carboxamide, d'un de ses sels pharmaceutiquement acceptables ou d'un de leurs solvats pour la préparation de médicaments utiles pour la prévention et pour le traitement de telles maladies.

Le composé A et ses sels pharmaceutiquement acceptables sont préparés selon le brevet européen EP 656 354, de même les compositions pharmaceutiques peuvent être préparées selon la description de ce même brevet.

Dans les compositions pharmaceutiques de la présente invention pour l'administration orale, sublinguale, sous-cutanée, intramusculaire, intraveineuse, transdermique, locale ou rectale, le principe actif seul ou en association avec un autre principe actif peut être administré sous forme unitaire d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les implants, les formes d'administration sous-cutanée, transdermique, intramusculaire, intraveineuse, intranasale et les formes d'administration rectale.

Le dosage journalier du composé selon l'invention est de 0,001 à 5 mg/kg, avantageusement de 0,01 à 2,5 mg/kg, préférentiellement de 0,02 à 2 mg/kg, à administrer en une ou plusieurs fois. Les composés sont généralement formulés en unité de dosage contenant de 0,1 à 500 mg, avantageusement de 1 à 250 mg, préférentiellement de 2 à 200 mg, de principe actif par unité de dosage, à administrer une fois, deux fois ou plusieurs fois en même temps, selon la nécessité. Bien que ces dosages soient des exemples de situation moyennes, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, l'âge, le poids et la réponse dudit patient.

Selon la présente invention, les formes orales d'administration sont préférées.

L'invention est également relative à une méthode de traitement des pathologies neuroinflammatoires, en particulier les maladies entraînant une démyélinisation telles que la sclérose en plaques ou le syndrome de Guillain-Barré par exemple ; ainsi que les encéphalites virales, les accidents vasculaires cérébraux ou les traumatismes crâniens.

L'activité de l'antagoniste des récepteurs aux cannabinoïdes CB₁ a été recherchée dans le modèle d'encéphalite allergique expérimentale (EAE) induite :
a) chez le rat Lewis par l'administration intraplantaire de protéine basique de myéline (MBP) (fragment 68-84) dans un adjuvant complet de Freund (FCA) enrichi en *mycobacterium tuberculosis* selon le protocole publié par Martin et Near (J. Neuroimmunol., 1995, 241-245),
b) chez la souris SjL/j par l'administration sous-cutanée de peptide protéolipide (PLP) (fragment 139-151) dans un adjuvant complet de Freud enrichi en *mycobacterium tuberculosis* selon le protocole décrit dans Proc. Natl. Acad. Sci. USA, 1996,. 93, 2499-2504. 24 et 48 heures après cette injection, les souris reçoivent une suspension de *Bordetella pertusis* par voie intraveineuse.

L'EAE est une maladie autoimmune et inflammatoire du système nerveux central présentant des lésions démyélinisantes rappelant par exemple celle de la sclérose en plaques humaine.

Dans les modèles expérimentaux, le composé représentatif de l'invention, administré par voie orale ou intrapéritonéale depuis le jour zéro d'induction de la maladie, atténue de façon très significative la maladie, atténuation mesurée à la fois sur les variations de poids des animaux (les animaux malades présentent une perte importante de poids) et sur la sévérité de la pathologie (les animaux malades présentent une paralysie des pattes postérieures). La perte de poids des animaux traités, par le composé A est significativement plus faible que celle des animaux traités par le seul véhicule. De même, la sévérité de la maladie est statistiquement plus faible dans les groupes d'animaux traités par le composé A.

Les résultats de ces études montrent que le composé A, antagoniste des récepteurs aux cannabinoïdes CB₁ et ses sels et solvats pharmaceutiquement acceptables, interviennent favorablement dans cette pathologie de dysfonctionnement neurologique et peuvent ainsi trouver une application clinique dans le traitement de maladies causant des lésions démyélinisantes, telle que la sclérose en plaques.

| EXEMPLE 1 Gélule dosée à 1 mg d'antagoniste des récepteurs CB1. | |
|---|---|
| Composé A micronisé | 1,00 mg |
| Amidon de maïs | 51,00 mg |
| Lactose monohydrate | 103,33 mg |
| Polyvidone | 4,30 mg |
| Sodium laurylsulfate | 0,17 mg |
| Carboxyméthyl cellulose de sodium réticulé | 8,50 mg |
| Eau purifiée : Q.S. pour granulation humide | |
| Stéarate de magnésium | 1,70 mg |
| Pour une gélule blanc opaque n° 3 remplie à 170 mg | |

| EXEMPLE 2 Gélule dosée à 10 mg d'antagoniste des récepteurs CB1. | |
|---|---|
| Composé A micronisé | 10,00 mg |
| Amidon de maïs | 51,00 mg |
| Lactose monohydrate | 94,33 mg |
| Polyvidone | 4,30 mg |
| Sodium laurylsulfate | 0,17 mg |
| Carboxyméthyl cellulose de sodium réticulé | 8,50 mg |
| Eau purifiée : Q.S. pour granulation humide | |
| Stéarate de magnésium | 1,70 mg |
| Pour une gélule blanc opaque n° 3 remplie à 170 mg | |

| EXEMPLE 3 Gélule dosée à 30 mg d'antagoniste des récepteurs CB1. | |
|---|---|
| Composé A micronisé | 30,00 mg |
| Amidon de maïs | 51,00 mg |
| Lactose monohydrate | 74,33 mg |
| Polyvidone | 4,30 mg |
| Sodium laurylsulfate | 0,17 mg |
| Carboxyméthyl cellulose de sodium réticulé | 8,50 mg |
| Eau purifiée : Q.S. pour granulation humide | |
| Stéarate de magnésium | 1,70 mg |

Pour une gélule blanc opaque n° 3 remplie à 170 mg

| EXEMPLE 4 Comprimé dosé à 30 mg d'antagoniste des récepteurs CB1. | |
|---|---|
| Composé A micronisé | 30,00 mg |
| Lactose monohydrate | Q.S. |
| Amidon de maïs | 40,00 mg |
| Hydroxypropylméthyl cellulose 6cP | 5,00 mg |
| Eau purifiée : Q.S. pour granulation humide | |
| Carboxyméthyl cellulose de sodium réticulé | 10,00 mg |
| Stéarate de magnésium | 2,00 mg |
| Pour un comprimé terminé à 200 mg. | |

## Revendications

1. Utilisation du N-pipéridino-5-(4-chlorophényl)-1-(2,4-dichlorophényl)-4-méthylpyrazole-3-carboxamide ou d'un de ses sels pharmaceutiquement acceptable ou d'un de leurs solvats pour la préparation de médicaments utiles pour prévenir ou traiter les maladies entraînant une démyélinisation, les encéphalites virales, les accidents vasculaires cérébraux ou les traumatismes crâniens.

2. Utilisation selon la revendication 1, pour la préparation de médicaments utiles pour prévenir ou traiter la sclérose en plaques.

## Patentansprüche

1. Verwendung von N-Piperidino-5-(4-chlorphenyl)-1-(2,4-dichlorphenyl)-4-methylpyrazol-3-carboxamid oder eines seiner pharmazeutisch annehmbaren Salze oder eines seiner Solvate für die Herstellung von Arzneimitteln für die Vorbeugung oder die Behandlung von Erkrankungen, die zu einer Demyelinisation führen, von Virus-Encephalitis, Schlaganfällen oder Schädeltraumata.

2. Verwendung nach Anspruch 1 für die Herstellung von Arzneimitteln für die Vorbeugung und die Behandlung von Multipler Sklerose.

## Claims

1. Use of N-piperidino-5-(4-chlorophenyl)-1-(2,4-dichlorophenyl)-4-methylpyrazole-3-carboxamide or of a pharmaceutically acceptable salt or of a solvate thereof for the preparation of medicinal products that are useful for preventing or treating conditions involving demyelinization, viral encephalitis, cerebrovascular accidents or cranial trauma.

2. Use according to Claim 1, for the preparation of medicinal products that are useful for preventing or treating multiple sclerosis.
